# FASCICULE DE BREVET EUROPEEN

(11) **EP 3 646 890 B1**
(45) Date de publication et mention de la délivrance du brevet: **19.06.2024**
(21) Numéro de dépôt: 19207232.0
(22) Date de dépôt: 05.11.2019
(51) Int. Cl.: A61L 2/00, C12Q 1/22, C12Q 1/02, C12N 7/06, A61L 2/10, A61L 2/08, A61L 2/28

(54) **BIO-INDICATEUR DE DÉCONTAMINATION À LUMIÈRE PULSÉE HAUTE FRÉQUENCE**
HOCHFREQUENTE GEPULSTE LICHTDEKONTAMINATION BIO-INDIKATOR
HIGH-FREQUENCY PULSED LIGHT DECONTAMINATION BIO-INDICATOR

(30) Priorité: 05.11.2018 FR 1871370
(43) Date de publication de la demande: 06.05.2020
(73) Titulaire: Sterixene, 30133 Les Angles (FR)
(72) Inventeur: FRANC, Janyce, 84000 Avignon (FR); PUISNEL, Christophe, 84000 Avignon (FR)
(74) Mandataire: Rhein, Alain

(56) Documents cités:
- EP-A1- 2 805 912
- WO-A1-02/092138
- WO-A1-2012/092684
- WO-A2-00/04930
- WO-A2-02/26270
- GB-A- 1 055 387
- US-A- 6 004 741
- US-A1- 2008 265 179
- ELMNASSER N ET AL: "Pulsed-light system as a novel food decontamination technology: a review", CANADIAN JOURNAL OF MICROBIOLOGY, NRC RESEARCH PRESS, CA, vol. 53, no. 7, 1 juillet 2007 (2007-07-01), pages 813-821, XP002496349, ISSN: 0008-4166, DOI: 10.1139/W07-042
- FINE F ET AL: "EFFICIENCY OF PULSED UV LIGHT FOR MICROBIAL DECONTAMINATION OF FOOD POWDERS", JOURNAL OF FOOD PROTECTION, INTERNATIONAL ASSOCIATION FOR FOOD PROTECTION, US, vol. 67, no. 4, 1 avril 2004 (2004-04-01), pages 787-792, XP009069752, ISSN: 0362-028X
- Riedel Christophe ET AL: "Pulsed-Light Entkeimung Robotic Tub Decontaminating System", 25.-26. September 2018, Wiesbaden/Niedernhausen, 25 September 2018 (2018-09-25), pages 1-44, XP055971176,
- CAROLINE LEVY ET AL: "Deposition ofspores using an airbrush-spray or spots to study surface decontamination by pulsed light", JOURNAL OF MICROBIOLOGICAL METHODS, ELSEVIER, AMSTERDAM, NL, vol. 84, no. 2, 24 November 2010 (2010-11-24), pages 223-227, XP028137121, ISSN: 0167-7012, DOI: 10.1016/J.MIMET.2010.11.021 [retrieved on 2010-11-30]
- BOURGET EMMANUELLE: "POINTS COMMONS ET DIFFERENCES DANS LA CONCEPTION DES INDICATEURS BIOLOGIQUES POUR BIO-DECONTAMINATIONSPAR VOIE CHIMIQUE ET PAR LUMIERE PULSEE", SÉMINAIRE AUDITS 11, 17 September 2002 (2002-09-17), pages 1-28, XP055971360,

## Description

La présente invention se rapporte à la décontamination de produit et de matériel dans les domaines de l'industrie agroalimentaire, pharmaceutique, cosmétique, médicale, aéronautique etc.

Plus précisément, la présente invention concerne les technologies de décontamination au rayonnement ultraviolet (UV) telles que la technologie UV LED, UV continus, Excimères et plus particulièrement la technologie de décontamination par lumière pulsée haute fréquence.

L'objet de l'invention fournit une solution de contrôle de l'efficacité de la décontamination UV et notamment par lumière pulsée haute fréquence.

Il convient de rappeler que la technologie de décontamination par lumière pulsée consiste à irradier des produits, des liquides, des environnements ou des objets à décontaminer par émission de lumière pulsée enrichie en rayonnement UV. Le document US 4,464,336 décrit les principes généraux de cette technologie de décontamination.

Bien que cette technologie ait fait ses preuves, lors de la mise en place d'un système de décontamination par lumière pulsée dans un processus industriel, il convient de s'assurer de l'efficacité de la décontamination afin d'optimiser les réglages du système (puissance et fréquence de la lampe UV, angle d'irradiation etc.). De la même manière, dans le cadre d'un entretien d'un système de décontamination, il est primordial de vérifier l'efficacité de la décontamination sur les objets, produits ou matériaux irradiés.

Un système de décontamination par lumière pulsée peut être mis en place sur une chaine de production agroalimentaire, pharmaceutique ou cosmétique en vue de décontaminer l'emballage et/ou le contenant d'un produit consommable.

A titre d'exemple, en matière de décontamination d'un dispositif médical, la décontamination est jugée stérile selon la norme EN556 si la probabilité théorique est égale ou inférieure à 10⁻⁶ qu'un micro-organisme vivant soit présent à la surface l'objet. Par ailleurs, cette probabilité théorique égale ou inférieure à 10⁻⁶ est qualifiée d'une décontamination à 6 log d'efficacité. Cette probabilité théorique est appelée le Niveau d'Assurance Stérilité ou NAS, il exprime une probabilité qu'un système soit non stérile.

Toutefois en pratique, il n'existe pas d'outil permettant de vérifier l'efficacité de la décontamination d'un dispositif médical établi par cette norme.

De plus, à ce jour, le contrôle de l'efficacité de la décontamination par rayonnement UV n'est pas encore réglementé.

Dans le milieu industriel, la plupart du temps le contrôle de l'efficacité de la décontamination par rayonnement UV est réalisé de façon artisanale. Plus particulièrement, une opération de contrôle est le plus souvent effectuée par insertion, sans précautions particulières, de micro-organismes sur un support destiné à être décontaminé. Le support portant les micro-organismes est ensuite exposé aux irradiations de l'installation de décontamination par rayonnement UV que l'on souhaite contrôler. Après irradiation, les micro-organismes sont collectés de manière à analyser la quantité de micro-organismes détruits et/ou vivants.

Ces pratiques présentent un risque important de contamination des éléments de l'installation de décontamination et de l'environnement dans lequel elle est installée. Afin de limiter le risque de contamination, les processus industriels sont stoppés le temps de réaliser les tests.

Dans le cas d'une chaine de production, l'arrêt de la production peut durer une journée entière.

En plus du manque à gagner important que représente l'arrêt des processus industriels, ce type de méthode fait courir un risque, paradoxal, de contamination du matériel industriel par les micro-organismes utilisés pour contrôler l'efficacité des installations de décontamination. En effet, l'arrêt des processus industriels lors des tests ne permet pas de limiter le risque de contamination du matériel qui se retrouve au contact des micro-organismes.

A noter, qu'il est connu par le document WO 02/26270 un procédé de décontamination à la lumière pulsée de poches de sang hermétique translucide. Le document WO 2012/092684décrit un bioindicateur comprenant une enveloppe microporeuse perméable à un agent stérilisant et imperméable aux micro-organismes qu'ils supportent. Il est également connu notamment par les publications :
- Riedel Christophe ET AL : « Pulsed-Light Entkeimung Robotic Tub Decontaminationg Sydtem », Aseptikon 25-26 September 2018, Wiesbaden/Niedernhausen,
- CAROLINE LEVY ET AL « Déposition ofspores using an airbush-spray or spots to study surface decontamination by pulsed light », JOURNAL OF MICROBIOLOGICAL METHODS, ELSEVIER, AMSTERDAM, NL vol. 84, no. 2, 24 novembre 2010, pages 223-227, ISSN : 0167-7012, DOI : 10.1016/J.MIMET. 2010.11.021 ;
un bio-indicateur de décontamination par une technologie de décontamination aux rayonnements UV par lumière pulsée, le bio-indicateur comprenant une boite de Pétri qui renferme un élément support supportant des micro-organismes.

La demanderesse a développé une solution technique permettant d'effectuer le contrôle de l'efficacité d'une installation de décontamination par rayonnement UV lors de son fonctionnement normal et sans risque de contamination du matériel de production.

La portée de la protection est définie seulement par les revendications annexées.

A cet effet, un premier aspect de la présente invention se rapporte à un bio-indicateur de décontamination par une technologie de décontamination aux rayonnements UV par lumière pulsée. Le bio-indicateur, selon l'invention, comprend un contenant qui renferme un élément support supportant un nombre déterminé de micro-organismes, l'élément support est un support solide et les micro-organismes sont disposés sur l'élément support selon un ensemencement en monocouche, le contenant comportant une enveloppe hermétique qui est formée par des parois, dont au moins une portion est transparente à la lumière pulsée selon un spectre de longueur d'onde compris entre 180 nm et 1100 nm, cette portion transparente étant disposée au regard de l'élément support afin que les micro-organismes puissent être tous directement exposés aux rayonnements de la lumière pulsée, l'enveloppe étant formée par au moins une feuille de matériau polymérique et/ou composite pliée en deux de manière à former les deux parois de l'enveloppe, afin que l'enveloppe soit hermétique la feuille repliée est au moins partiellement scellée sur le pourtour de l'enveloppe, ou par deux feuilles de matériau polymérique et/ou composite formant deux parois de l'enveloppe, les deux feuilles étant scellées entre elles sur leur pourtour.Le caractère hermétique et la transparence spectrale à la lumière pulsée contribuent à fournir un bio-indicateur assurant un contrôle sécurisé et précis de l'efficacité de fonctionnement d'une installation de décontamination au rayonnement UV par lumière pulsée sans stopper le processus industriel. L'industriel gagne ainsi en rendement et en sécurité lors des contrôles d'efficacité.

Selon une première caractéristique du premier aspect de l'invention, l'enveloppe est entièrement transparente à la lumière pulsée selon un spectre de longueur d'onde compris entre 180 nm et 1100 nm, de préférence l'enveloppe est réalisée dans un matériau polymérique et/ou composite présentant des propriétés optiques de transparence à la lumière selon un spectre de longueur d'onde compris entre 180 nm et 1100 nm. Selon une première possibilité de réalisation de l'invention, l'enveloppe est formée par au moins une feuille de matériau polymérique et/ou composite pliée en deux de manière à former les deux parois de l'enveloppe, la feuille repliée est au moins partiellement scellée sur son pourtour. Selon une deuxième possibilité de réalisation de l'invention, l'enveloppe est formée par deux feuilles de matériau polymérique et/ou composite formant deux parois de l'enveloppe, les deux feuilles étant scellées entre elles sur leur pourtour.

Selon une deuxième caractéristique du premier aspect de l'invention, l'élément support est au moins partiellement hydrophile.

Selon une troisième caractéristique du premier aspect de l'invention, les micro-organismes sont maintenus en suspension dans une solution aqueuse non saline. Le caractère non salin, évite la formation d'une couche de sel qui diminuerait l'exposition des micro-organismes contenus dans la solution aqueuse. Ainsi, cette caractéristique contribue à optimiser l'exposition de la totalité des micro-organismes contenus dans le bio-indicateur aux rayonnements de la lumière pulsée. De fait, la représentativité du contrôle d'efficacité du fonctionnement de l'installation de décontamination se voit améliorée.

Selon une quatrième caractéristique du premier aspect de l'invention, . le support solide est formé, par au moins une partie d'une face intérieure d'une paroi de l'enveloppe, ou par une plaque solide contenue par l'enveloppe.

Selon une cinquième caractéristique du premier aspect de l'invention, les micro-organismes sont disposés sur l'élémen support selon un ensemencement en monocouche. Cette caractéristique évite une superposition des micro-organismes et contribue à obtenir, une exposition aux rayonnements de la lumière pulsée, de la totalité des micro-organismes contenus dans le bio-indicateur. De fait, la précision du contrôle d'efficacité du fonctionnement de l'installation de décontamination est optimisée.

Selon une sixième caractéristique du premier aspect de l'invention, l'élément support est formé de préférence par matériau qui possède une énergie de surface comprise entre 35 mN/m et 55 mN/m, de préférence, l'élément support est formé par un matériau qui possède une énergie de surface comprise entre 40 mN/m et 50 mN/m, et de préférence l'élément support est formé par un matériau qui possède une énergie de surface comprise entre 43 mN/m et 48 mN/m. Avantageusement, lorsque l'élément support possède ces propriétés d'énergie de surface, cela favorise un étalement de la goutte de solution aqueuse non saline comprenant les micro-organismes contribuant à l'obtention d'une répartition monocouche des micro-organismes.

L' élément support pourrait être un support liquide. Dans ce cas, le support liquide est constitué par une solution aqueuse dans laquelle les micro-organismes sont en suspension, la solution aqueuse est non saline. Le caractère non salin contribue comme évoqué plus haut à optimiser l'exposition des micro-organismes aux rayonnements de la lumière pulsée.

Selon une septième caractéristique du premier aspect de l'invention, le bio-indicateur comporte des moyens de fixation à une surface d'accroche.

Un deuxième aspect de l'invention porte sur un procédé de préparation d'un bio-indicateur conforme au premier aspect de l'invention.

Le procédé de préparation se caractérise en ce qu'il comporte :
- une étape de stérilisation de l'élément support ;
- une étape d'ensemencement de l'élément support par des micro-organismes ;
- une étape de formation de l'enveloppe ; et
- une étape de scellement hermétique de l'enveloppe.

Un troisième aspect de l'invention concerne un procédé de contrôle de l'efficacité d'un système de décontamination par une technologie de décontamination aux rayonnements UV et notamment de la technologie de décontamination par lumière pulsée.

Le procédé de contrôle utilise un bio-indicateur conforme au premier aspect de l'invention, le procédé de contrôle se caractérise en ce qu'il comporte :
- une étape d'accrochage d'au moins un bio-indicateur qui est solidarisé à un élément prenant part à un processus industriel soumis à une décontamination à la lumière pulsée ;
- une étape d'irradiation qui consiste à exposer au moins un bio-indicateur positionné sur ledit élément aux rayonnements de la lumière pulsée ; et
- une étape d'analyse de la quantité de micro-organismes détruits et/ou vivants, suite à l'irradiation, l'étape d'analyse comportant une opération de récupération des micro-organismes vivants et une opération de culture des micro-organismes vivants afin de déterminer l'efficacité du système de décontamination.

D'autres particularités et avantages apparaitront dans la description détaillée, qui suit, de deux exemples de réalisation, non limitatifs, de l'invention qui sont illustrés par les figures 1 à 5 placées en annexe et dans lesquelles :
- la figure 1 est une représentation éclatée en perspective d'un bio-indicateur conforme à un premier exemple de réalisation de l'invention ;
- la figure 2 est une représentation éclatée en perspective d'un bio-indicateur conforme à une variante du premier exemple de réalisation de l'invention ;
- la figure 3 est une représentation en perspective d'un bio-indicateur conforme à un second exemple de réalisation de l'invention ;
- la figure 4 est un diagramme de représentation du spectre de transmission électromagnétique d'un exemple de composé fluoro-éthylène ; et
- la figure 5 est un diagramme de représentation du spectre de transmission électromagnétique d'un exemple de quartz synthétique.

La présente invention se rapporte à un bio-indicateur 1 de décontamination par technologies de décontamination UV, et plus particulièrement par la décontamination à la lumière pulsée haute fréquence.

Le bio-indicateur 1 selon l'invention est configuré pour contrôler l'efficacité de décontamination d'une technologie de décontamination à la lumière pulsée.

A cet effet, le bio-indicateur 1 est configuré pour être utilisé directement sur les lignes de production en fonctionnement normal.

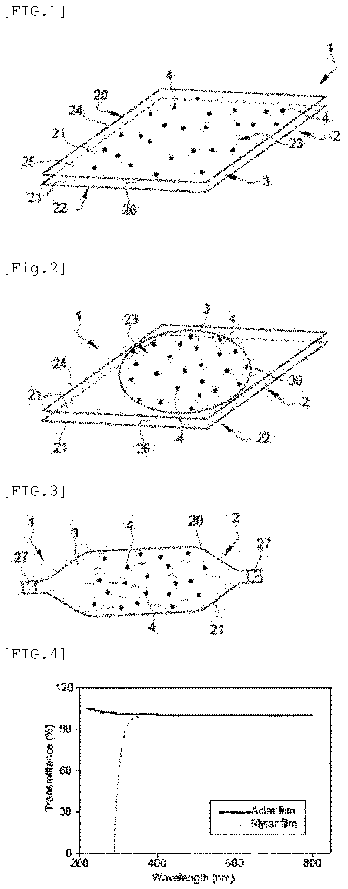

Comme illustré aux figures 1 à 3, le bio-indicateur 1 comprend un contenant 2 qui renferme un élément support 3 de micro-organismes 4. Afin de supporter les micro-organismes 4, l'élément support 3 est au moins partiellement hydrophile. Classiquement, les micro-organismes 4 sont maintenus dans l'eau. Dans ce cadre, l'élément support 3 est au moins partiellement hydrophile, favorisant l'adhérence des micro-organismes 4.

L'élément support 3 peut être formé par un support solide ou par un support liquide. Ici, le terme solide est employé au sens de l'état physique solide. C'est-à-dire, un état de la matière dans lequel les atomes oscillent autour de positions fixes selon une distribution arbitraire (solides amorphes) ou ordonnée (cristaux).

Plus particulièrement, le contenant 2 comporte une enveloppe 20 dans laquelle est contenue l'élément support 3 des micro-organismes 4. L'enveloppe 20 est délimitée par des parois 21 qui emprisonnent l'élément support 3. Plus précisément, l'enveloppe 20 est fermée de manière hermétique.

Le caractère hermétique de l'enveloppe 20 permet de contenir de manière sécurisée les micro-organismes 4. Il est ainsi possible d'éviter la contamination du matériel technique des chaînes de production. Cette caractéristique contribue à simplifier le procédé de contrôle de l'efficacité d'un système de décontamination par une technologie de décontamination UV. En effet, cela permet notamment de réaliser les tests d'efficacité sans pour autant stopper le fonctionnement des processus industriels.

A cet effet, l'enveloppe 20 peut comporter des moyens de fixation 22 à une surface d'accroche. De préférence, les moyens de fixation sont disposés au niveau de la surface extérieure des parois 21 de l'enveloppe 20.

La surface d'accroche peut être un élément technique ou une surface prenant part dans un processus industriel qui sont exposés aux rayonnements de la lumière pulsée. Dans le cas d'une chaîne de production, la surface d'accroche peut être un élément technique de la chaine de production ou un objet à décontaminer passant sur cette même chaine de production.

Les moyens de fixation 22 peuvent être de toutes natures, mécaniques ou chimiques. De préférence, les moyens de fixation 22 sont formés par un adhésif disposé au niveau de la surface extérieure des parois 21.

Comme cela est décrit dans la littérature scientifique, la lumière pulsée haute fréquence présente la particularité de fournir un flash intense, d'une puissance pouvant aller jusqu'à environ 2 MW, de lumière enrichie en rayonnements ultraviolet ou UV. En effet, il est connu que la lumière pulsée comportant un spectre lumineux composé de 20% de rayonnements UV, 50% de rayonnement de la lumière visible, et 30% de rayonnements infrarouge proche.

Contrairement à la décontamination par UV continue qui n'utilise qu'une seule longueur d'onde d'émission d'une valeur de 254 nm, la décontamination par lumière pulsée haute fréquence émet sur un large spectre lumineux allant de 180 nm à 1100 nm. Il est par ailleurs admis que les rayonnements compris dans le spectre ultraviolet sont les rayonnements les plus germicides.

Ainsi, l'enveloppe 20 possède au moins une portion 23 qui est transparente au moins aux rayonnements UV. Toutefois, afin que le test d'efficacité de la technologie de décontamination à lumière pulsée soit optimisé, la portion 23 est transparente à tout le spectre électromagnétique de la lumière pulsée. Cette dernière est la technologie de décontamination UV qui présente le plus large spectre de rayonnement.

Cette portion 23 transparente est disposée au regard de l'élément support 3. Cet agencement contribue à ce que tous les micro-organismes 4 disposés sur l'élément support 3 puissent être directement exposés aux rayonnements de la lumière pulsée.

De préférence, l'enveloppe 20 est entièrement transparente aux rayonnements de la lumière pulsée et notamment aux rayonnements UV.

A cet effet, l'enveloppe 20 peut être réalisée dans un matériau synthétique qui présente des propriétés optiques de transparence selon un spectre de longueur d'onde compris entre 170 nm et 2680 nm. Avantageusement, cette plage de valeurs du spectre couvre la plage de valeur des rayonnements émis par la lumière pulsée.

A titre indicatif, le matériau synthétique peut être de nature polymérique/composite. Plus spécifiquement, il est possible de choisir ce matériau parmi le groupe des composés polymériques fluorés et plus particulièrement des composés polymériques présentant un motif fluoré à chaîne courte. Les fluoro-éthylènes sont un exemple de ce type de composés, et plus précisément un polymère de poly-chloro-trifluoro-éthylène (PCTFE) présente de telles propriétés optiques.

La figure 4 illustre un exemple de spectre de transmittance du PCTFE sous la dénomination commerciale « ACLAR ». Ce matériau présente avantageusement une transmittance supérieure à 90% pour des valeurs de longueurs d'onde allant du 220 nm à 800 nm.

Il est également possible de réaliser l'enveloppe 20 dans un matériau synthétique de type quartz synthétique à partir de silice fondue synthétique. Le matériau commercialisé sous la marque « Suprasil » est un exemple de ce type de quartz synthétique. Avantageusement, les matériaux de type quartz synthétique présentent des propriétés optiques remarquables et notamment une transmittance de l'ordre de 90% pour des valeurs de longueurs d'onde comprise entre 180 nm et 2660 nm. Or, ces propriétés de transmittance sur cette plage de valeurs permettent de couvrir le spectre d'émission de la lumière pulsée et plus particulièrement des rayonnements UV de la lumière pulsée qui se situent entre 180 nm et 400 nm.

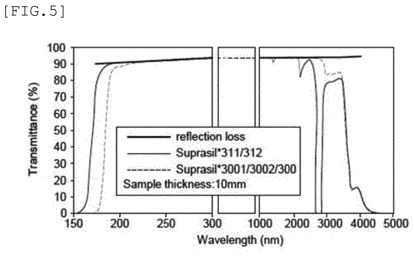

En ce sens, la figure 5 illustre, pour plusieurs matériaux de type quartz synthétique, le pourcentage de transmission des rayonnements électromagnétiques en fonction de leurs longueurs d'onde.

Ces deux exemples de type de matériau permettent la transmission de l'intégralité du spectre d'émission de la lumière pulsée au travers de l'enveloppe 20. Les micro-organismes 4 contenus par le bio-indicateur 1 sont ainsi exposés de manière efficace aux irradiations du rayonnement de la lumière pulsée. In fine, il est possible de déterminer précisément l'efficacité d'une installation de décontamination à la lumière pulsée haute fréquence.

Dans cet objectif, le bio-indicateur 1 comporte un nombre déterminé de micro-organismes 4. En pratique, le bio-indicateur 1 comporte un ordre de grandeur déterminé de micro-organismes 4. Cet ordre de grandeur varie en fonction de la puissance du système de décontamination à la lumière pulsée dont on souhaite déterminer l'efficacité.

De manière générale, l'efficacité d'une décontamination s'exprime en log. A titre d'exemple, si une division par 1000 du nombre de micro-organismes 4 est déterminée après décontamination, cette décontamination est dite de 3 log. Une division par 10 000 du nombre de micro-organismes 4 est-elle dite de 4 log etc.

Comme l'illustre le tableau 1 ci-dessous, le bio-indicateur 1 selon l'invention est prévu de manière à prouver jusqu'à une efficacité de 6 log. Ainsi, en fonction de l'efficacité que l'on souhaite démontrer, l'élément support 3 est ensemencé du nombre micro-organismes 4 adéquats (voir Tableau 1).

**Tableau 1**

| Efficacité décontamination (en log) | Nombre de micro-organismes |
|---|---|
| 3 | 2000 à 7000 |
| 4 | 20 000 à 70 000 |
| 5 | 200 000 à 700 000 |
| 6 | 2 000 000 à 7 000 000 |

En outre, les micro-organismes 4 peuvent comprendre un nombre déterminé de bactéries, de champignons, de virus, de parasites etc.

Tous types de micro-organisme 4 peuvent être employés afin de démontrer l'efficacité de la décontamination par rayonnement UV.

Toutefois, la bactérie de la famille des Bacilles, *Bacillus atrophaeus* DSM 675 spores, a historiquement été utilisée comme bio-indicateur pour évaluer l'efficacité de la décontamination chimique et thermique. Il est à noter qu'il est également possible d'utiliser *Bacillus subtilis* ATCC 633 ou *Bacillus cereus ATCC 14579 ou Bacillus pumilus DSM 492 ou Geobacillus stearothermophillus ATCC 7953.*

Toutefois, tout type de bactérie peut être utilisé pour évaluer l'efficacité d'une décontamination par rayonnement UV.

De la même manière, le champignon *Aspergillus brasiliensis DSM 1988 ou Aspergillus brasiliensis ATCC 16404* spores est habituellement utilisé pour évaluer l'efficacité de la décontamination chimique et/ou thermique d'un objet. Cependant, tout type de champignon peut être utilisé pour évaluer l'efficacité d'une décontamination par rayonnement UV.

Que l'élément support 3 soit solide ou liquide, les micro-organismes 4 sont maintenus en suspension dans une solution aqueuse non saline.

Dans le cas d'un support solide, les micro-organismes 4 sont déposés au travers d'une goutte de solution aqueuse non saline.

Le caractère non salin de la solution aqueuse permet de prévenir toute cristallisation de sels. Ce phénomène de cristallisation saline est d'autant plus important, lorsqu'on réalise une opération de séchage de l'échantillon déposé à la surface de l'élément support 3. En effet, la cristallisation de sels protège les micro-organismes 4 des rayonnements de la lumière pulsée et biaise les résultats du contrôle de l'efficacité d'une installation de lumière pulsée.

Ce phénomène est illustré par le tableau 2 ci-dessous qui correspond à des résultats d'expérience qui visent à déterminer l'efficacité de la décontamination à la lumière pulsée en fonction de la salinité de la solution aqueuse dans laquelle les micro-organismes sont contenus. Ces expériences ont été réalisées à l'aide d'un bio-indicateur 1 à support solide implémenté d'une goutte de 10µl soit 4mm de diamètre possédant des micro-organismes 4 de type *Aspergillus brasiliensis* selon un ensemencement initial de 3 Log.

Ces expériences ont par ailleurs été menées à l'aide d'une lampe de lumière pulsée disposée à 58 mm de chaque bio-indicateur, ladite lampe ayant produit deux flashs de 1,4 J/cm².

**Tableau 2**

| Teneur en sel de la solution aqueuse (en g/l) | Efficacité de décontamination (en Log) |
|---|---|
| 0 | 2,2 |
| 4 | 1,6 |
| 6 | 1,4 |
| 8,5 | 0,7 |

Les résultats d'expérience exposés dans le Tableau 2, montrent que plus la solution aqueuse, dans laquelle les micro-organismes sont en suspension, est concentrée en sels, plus l'efficacité de la décontamination par lumière pulsée diminue. Ainsi, pour une solution aqueuse possédant une concentration en sel de 8,5 g/l, il est observé une efficacité réduite à 0,7 Log. A l'inverse, lorsque la concentration en sel de la solution aqueuse est nulle, il est observé une efficacité de décontamination de 2,2 Log.

Selon un premier exemple de réalisation de l'invention illustré aux figures 1 et 2, l'enveloppe 20 est délimitée par des bordures latérales périphériques 25 qui délimitent le pourtour de l'enveloppe 20. En outre, l'enveloppe 20 comporte des parois 21 qui s'étendent parallèlement l'une à l'autre entre les bordures latérales périphériques 24. Dans cet exemple, les parois 21 forment deux faces extérieures 25 de l'enveloppe 20 du bio-indicateur 1.

Dans cet exemple, l'enveloppe 20 est de forme rectangulaire, toutefois, l'enveloppe 20 peut prendre toute sorte de formes géométriques. En effet, la forme géométrique de l'enveloppe 20 peut varier en fonction de l'environnement dans lequel le bio-indicateur 1 va être utilisé.

Les dimensions du bio-indicateur 1 et donc de son enveloppe 20 peuvent varier en fonction de la destination du bio-indicateur 1 et du niveau d'efficacité que l'on souhaite évaluer (nombre de micro-organisme 4 ensemencés). Ainsi, le bio-indicateur 1 peut avoir une taille comprise entre 0,5 cm² et 100 cm².

Comme illustré aux figures 1 et 2, l'enveloppe 10 est formée par au moins une feuille d'un matériau adapté tel que décrit précédemment. En effet, il est possible de former une enveloppe 20 en pliant une feuille d'un matériau adapté en deux. Les deux parties de la feuille situées de part et d'autre de la pliure constituant une paroi 21 de l'enveloppe 20. Afin que l'enveloppe 20 soit hermétique, la feuille repliée est au moins partiellement scellée sur le pourtour de l'enveloppe 20.

Dans cet exemple, l'enveloppe 20 est formée par deux feuilles de matériau adaptées qui forment respectivement deux parois 21 de l'enveloppe 20. Le caractère hermétique de l'enveloppe 20 est obtenu en scellant les deux feuilles entre elles sur le pourtour de l'enveloppe 20.

Selon les propriétés du matériau adapté, il est possible de réaliser l'opération de scellage par thermo-soudure. C'est notamment le cas lorsque des feuilles de la famille des polymères fluorés précités sont utilisées pour réaliser l'enveloppe 20.

Dans l'exemple de la figure 1, l'élément support 3 est un support solide. Ici, l'élément support 3 est constitué par au moins une partie d'une face intérieure 26 d'une paroi 21 de l'enveloppe 20. Selon cet exemple, la partie de la face intérieure 26 qui accueille les micro-organismes 4 est au moins partiellement hydrophile.

Il est à noter qu'il est possible de polariser la surface d'un matériau pour le rendre partiellement hydrophile. Cette polarisation peut être effectuée par un traitement physico/chimique de surface. A titre indicatif, il est possible de polariser un matériau polymérique fluoré précité au travers d'un traitement de surface par « effet Corona ».

Dans cet exemple, les micro-organismes 4 sont disposés sur l'élément support 3 selon un ensemencement en monocouche. L'ensemencement en monocouche de l'élément support 3 contribue à améliorer la précision du procédé de contrôle de l'efficacité d'un système de décontamination à lumière pulsée. De plus, le paramètre d'ensemencement monocouche des micro-organismes 4 contribue à mettre en place un procédé de contrôle de l'efficacité fiable en assurant la répétabilité des résultats. En effet, un tel ensemencement assure que tous les micro-organismes 4 contenus par le bio-indicateur 1 soient exposés directement aux irradiations de la lumière pulsée.

Afin d'illustrer l'avantage de l'ensemencement monocouche, le tableau 3, ci-après, expose des résultats d'efficacité de décontamination par lumière pulsée en fonction de la concentration en micro-organismes 4 par rapport au volume de solution aqueuse contenant les micro-organismes 4.

A cet effet, un bio-indicateur 1 à support solide a été implémenté des micro-organismes 4 de type *Aspergillus brasiliensis* selon un ensemencement initial de 5 log. Par ailleurs, le volume de la solution aqueuse varie selon les échantillons entre 10 µL et 100 uL.

Concernant les échantillons de 100 uL, deux types de dépôt sont réalisés sur le support solide. Un premier type, dit « single spot » utilise le dépôt d'une seule goutte à la surface du support solide. Alors que le second type dit « multi-spot » consiste à déposer selon plusieurs gouttes à différents points d'un support un échantillon de volume déterminé.

Il est à noter que l'échantillon de 10µL est effectué sur selon la technique « single spot ».

Ces expériences ont par ailleurs été menées à l'aide d'une lampe de lumière pulsée disposée à 58 mm de chaque bio-indicateur, ladite lampe ayant produit quatre flashs de 1,2 J/cm².

**Tableau 3**

| Volume solution aqueuse (en µL) | Type de dépôt | Efficacité de décontamination (en Log) |
|---|---|---|
| 10 | Single spot | 0,9 |
| 100 | Single spot | 4 |
| 100 | Multi-spot | 2.9 |

Les résultats exposés par le tableau 3 montrent la meilleure efficacité de décontamination est obtenue pour un échantillon de 100 µL selon une répartition single spot sur le support solide. Ceci indique que le taux de concentration en micro-organisme 4 de l'échantillon influe sur l'efficacité de la décontamination par lumière pulsée. Cela s'explique par une répartition monocouche des micro-organismes 4 sur le support solide.

Il est à noter que l'échantillon de 100 µL selon une répartition multi-spot affiche une décontamination de 2,90 Log qui est inférieure de plus d'une unité log par rapport aux résultats de décontamination de l'échantillon de 100uL « single spot ». Ceci s'explique par le fait que la concentration en micro-organismes 4 n'est pas identique dans chaque goutte déposée et que certaines gouttes peuvent présenter une concentration de micro-organisme qui implique un ensemencent multicouche des micro-organismes. Or, comme évoqué au préalable, un ensemencement multicouche ne permet pas, d'exposer directement, tous les micro-organismes contenus par le bio-indicateur 1, aux rayonnements de la lumière pulsée.

En conséquence, l'invention utilise de préférence un ensemencement de micro-organismes 4 single spot dans un volume déterminé de solution aqueuse non saline.

La figure 2 illustre une variante de réalisation de l'invention dans laquelle, l'élément support 3 est également un support solide. Plus spécifiquement, l'élément support 3 est formé par une plaque 30 contenue par l'enveloppe 20. La plaque 30 s'étend longitudinalement entre les parois 21 de l'enveloppe 20. La plaque 30 est maintenue en position par le pourtour thermo-soudé de l'enveloppe 20. Avantageusement, la plaque 30 est de couleur blanche afin de refléter l'intégralité du spectre électromagnétique émis par l'installation de décontamination à la lumière pulsée.

La plaque 30 peut être réalisée dans un matériau métallique. Toutefois, la plaque 30 peut être réalisée dans un matériau métallique et/ou matériau polymérique et/ou composite. De préférence, la plaque 30 est réalisée dans un matériau polymérique et/ou composite qui est au moins partiellement hydrophile de nature. Par exemple, les composés de la famille des polyamides ou « PA » fournissent de telles caractéristiques. Plus particulièrement, il est possible d'utiliser du PA 6 ou du PA 6-6.

Le caractère hydrophile de l'élément support 3 contribue à optimiser l'étalement de la solution aqueuse contenant les micro-organismes 4 à la surface de l'élément support 3. De fait, le caractère hydrophile de l'élément support 3 favorise l'ensemencement monocouche des micro-organismes 4.

De préférence, l'élément support 3 peut être formé par un matériau qui possède une énergie de surface comprise entre 35 mN/m et 55 mN/m. De préférence, l'élément support 3 est formé par un matériau qui possède une énergie de surface comprise entre 40 mN/m et 50 mN/m. Et encore de préférence, l'élément support 3 est formé par un matériau qui possède une énergie de surface comprise entre 43 mN/m et 48 mN/m. Il est à noter que des matériaux comme le PA 6-6 possèdent une telle propriété. Les matériaux comme l'aluminium, l'acier ou le verre possèdent une énergie de surface supérieure à 500 mN/m. Toutefois, le niveau de l'énergie de surface peut être très affecté par le nettoyage ou l'état de surface (rayures). Par ailleurs, le métal peut présenter un risque pour les lignes de production en cas d'oxydation. En ce sens, il serait possible d'utiliser l'acier inoxydable poli pour former l'élément support 3. L'élément support métallique pourrait être réalisé en acier inoxydable poli avec une énergie de surface supérieure à 500 mN/m.

En revanche, les matériaux polymériques tels que le PA 6-6 sont inertes et ne présentent pas de risques pour les lignes de production. De plus, les matériaux polymériques sont très représentatifs des matériaux qui sont le plus souvent décontaminés à la lumière pulsée. A l'inverse, le verre présente un risque pour les lignes de production en cas de casse.

Selon un second exemple de réalisation de l'invention illustré à la figure 3, l'élément support 3 est constitué par un support liquide. En particulier, ici l'élément support 3 est composé d'une solution aqueuse. De préférence, cette solution aqueuse est non saline. Dans cet exemple, les microorganismes 4 sont en suspension dans la solution aqueuse.

Dans cet exemple, les parois 21 de l'enveloppe 20 sont réalisées dans un matériau rigide de type quartz synthétique tel que précédemment décrit. Ici, l'enveloppe 20 se présente sous la forme d'un récipient de type ampoule. Les parois de l'enveloppe 20 s'étendent entre deux extrémités 27. Au moins une de ces extrémités 27 est sécable afin d'extraire le contenu du bio-indicateur 1 après exposition aux irradiations d'une installation de décontamination à la lumière pulsée.

Toutefois, les parois 21 de l'enveloppe 20 peuvent également être constituées par au moins une feuille d'un matériau synthétique adéquat tel que décrit précédemment. L'enveloppe 20 s'apparente alors à une poche hermétique remplie d'une solution aqueuse non saline.

L'élément support 3 est choisi de nature liquide dans le cas où une installation de décontamination par rayonnement UV est conçue pour décontaminer un liquide. Ainsi, le bio-indicateur 1 traverse la zone d'exposition aux rayonnements UV tel que le fait le flux liquide à décontaminer. Le bio-indicateur 1 est ensuite récupéré pour analyse.

L'invention concerne également un procédé de préparation d'un bio-indicateur 1 de décontamination tel que décrit précédemment.

Le procédé de préparation d'un bio-indicateur 1 possède une étape de stérilisation de l'élément support 3.

Lorsque l'élément support 3 est un support solide, l'étape de stérilisation est réalisée à l'aide d'une enceinte à oxyde d'éthylène. Cependant, il est possible d'utiliser d'autres techniques de stérilisation dès lors qu'elles ne nécessitent pas une montée en température se rapprochant de la température de fusion du matériau utilisé pour concevoir l'élément support 3.

Lorsque l'élément support 3 est liquide, il est possible d'utiliser un liquide préalablement stérilisé ou de le stériliser à l'aide d'une technique adéquate (lumière pulsée ou rayonnement Gamma).

Le procédé de préparation d'un bio-indicateur 1 comprend, en outre, une étape d'ensemencement de l'élément support 3 par des micro-organismes 4. L'ensemencement consiste à charger l'élément support 3 d'un nombre déterminé de micro-organismes 4. Le nombre de micro-organismes 4 est déterminé en fonction de l'efficacité de la décontamination que l'on souhaite mesurer (voir tableau 1 ci-dessus).

Lorsque l'élément support 3 est un support solide, l'ensemencement des micro-organismes 4 est effectué en monocouche. A cet effet, il est possible d'utiliser plusieurs techniques d'ensemencement décrites par la littérature scientifique.

A titre d'exemple, un ensemencement par spot à la pipette graduée peut permettre d'obtenir un ensemencement en monocouche. Toutefois, il est également possible d'utiliser une méthode par spray ou de dépôt de gouttelettes avant un étalement à un outil d'étalement adapté tel qu'une cuillère.

Lorsque l'élément support 2 est un support liquide, l'étape d'ensemencement consiste à insérer un nombre déterminé de micro-organismes 4 dans la solution liquide choisie. En pratique, on utilise une solution aqueuse non saline.

De façon optionnelle, le procédé de préparation d'un bio-indicateur 1 peut comporter une étape de vérification de l'ensemencement en monocouche des micro-organismes 4. Cette vérification peut être effectuée à l'aide d'un microscope électronique à balayage ou MEB.

Lorsque l'élément support 3 est solide, le procédé de préparation d'un bio-indicateur 1 peut comprendre une étape de séchage de l'élément support 3 ensemencé. Cette étape consiste à réduire le volume de solution aqueuse dans lequel les micro-organismes 4 sont contenus. Cette étape est réalisée de manière classique.

Le procédé de préparation d'un bio-indicateur 1 comprend également une étape de formation de l'enveloppe 20.

Selon une première possibilité, lorsque l'élément support 3 est une plaque 30, l'élément support 3 ensemencé est positionné sur une feuille d'un matériau synthétique adapté tel que décrit précédemment, l'enveloppe 20 est alors formée par pliage de ladite feuille ou assemblage avec une seconde feuille d'un matériau adapté tel que décrit ci-dessus.

Selon une seconde possibilité, lorsque l'élément support 3 est constitué par une face intérieure 26 d'une paroi de l'enveloppe 20. Dans ce cas, une face d'une feuille d'un matériau adapté qui a été rendu au préalable au moins partiellement hydrophile et par la suite ensemencée est pliée en deux ou assemblée avec une seconde feuille de matériau adapté.

Selon une troisième possibilité, lorsque l'élément support 3 est liquide, l'enveloppe 20 est partiellement formée par pliage ou assemblage de feuilles de matériau adapté ou par soufflage d'un matériau de type quartz synthétique. Selon cette possibilité, le liquide constituant l'élément support 3 peut être ensemencé avant d'être placé dans l'enveloppe 20 partiellement fermée ou après avoir été placé dans l'enveloppe 20 au moins partiellement fermée.

Le procédé de préparation d'un bio-indicateur 1 possède également une étape de scellement hermétique de l'enveloppe 20. Cette étape peut être réalisée par thermo-soudure du pourtour de l'enveloppe 20 ou d'une extrémité 27 de l'enveloppe 20. Il est également possible de sceller l'enveloppe 20 par thermocollage ou par collage à froid.

Il est à noter qu'il est également possible de placer l'enveloppe 20 du bio-indicateur 1 sous vide lors de l'étape de scellement.

Le procédé de préparation peut également comporter une étape de stérilisation de la face extérieure des parois 21 de l'enveloppe 20 du bio-indicateur 1 avant son utilisation. Dans ce cas, afin de ne pas fausser les résultats de la décontamination par rayonnement UV, il est préférable d'utiliser des techniques de décontamination chimiques.

Cette opération permet d'obtenir un bio-indicateur 1 stérile utilisable dans une enceinte stérile. Une telle propriété peut, par exemple, permettre d'utiliser le bio-indicateur 1 pour contrôler l'efficacité de la décontamination par rayonnements UV de dispositifs médicaux qui est réalisée dans une enceinte stérile. De manière classique, après stérilisation de son enveloppe 20, le bio-indicateur 1 est stocké dans un emballage stérile.

De surcroît, l'invention se rapporte également à un procédé de contrôle de l'efficacité d'un système de décontamination à lumière pulsée haute fréquence utilisant un bio-indicateur 1 conforme à la présente invention.

Le procédé de contrôle selon l'invention comprend une étape d'accrochage. Lors de cette étape au moins un bio-indicateur 1 préalablement préparé est solidarisé à une surface d'accrochage. Le bio-indicateur 1 est fixé à cet élément à l'aide de ses moyens de fixation 22. En pratique, la surface d'accrochage peut être constituée par un élément prenant part à un processus industriel soumis à une étape de décontamination à la lumière pulsée. Bien entendu, le bio-indicateur 1 est positionné de manière à être exposé à lumière pulsée.

Le procédé de contrôle comporte une étape d'irradiation. L'étape d'irradiation consiste à exposer au moins un bio-indicateur 1 positionné sur ledit élément aux rayonnements de la lumière pulsée.

Avantageusement, de par l'utilisation d'un bio-indicateur 1 présentant un caractère hermétique, il est possible de réaliser l'opération d'irradiation sans arrêter le processus industriel. Ce qui représente un gain de temps et de production pour l'industriel.

Le procédé de contrôle comprend également une étape d'analyse de la quantité de micro-organismes 4 détruits et/ou vivants.

Suite à l'étape d'irradiation, le bio-indicateur 1 est collecté afin de réaliser une opération de récupération des micro-organismes 4 vivants ou morts.

De plus, aux fins de les compter, une opération de culture des micro-organismes 4 vivants est réalisée. L'opération de culture peut être réalisée de manière classique par une incubation dans une étuve des micro-organismes 4 vivants sur un milieu nutritif.

L'opération de culture est suivie d'un comptage du nombre de micro-organismes 4 afin de déterminer l'efficacité du système de décontamination.

Avantageusement, le résultat obtenu permet d'évaluer l'efficacité de l'installation de décontamination à la lumière pulsée testée. Cette évaluation peut permettre d'adapter les réglages de l'installation de décontamination afin d'en optimiser le fonctionnement.

## Revendications

1. Bio-indicateur (1) de décontamination par une technologie de décontamination aux rayonnements UV par lumière pulsée, le bio-indicateur (1) comprenant un contenant (2) qui renferme un élément support (3) supportant un nombre déterminé de micro-organismes (4), l'élément support (3) est un support solide et les micro-organismes (4) sont disposés sur l'élément support (3) selon un ensemencement en monocouche, le contenant (2) comportant une enveloppe (20) hermétique qui est formée par des parois (21), dont au moins une portion (23) est transparente à la lumière pulsée selon un spectre de longueur d'onde compris entre 180 nm et 1100 nm, cette portion (23) transparente étant disposée au regard de l'élément support (3) afin que les micro-organismes (4) puissent être tous directement exposés aux rayonnements de la lumière pulsée, l'enveloppe (20) étant formée par au moins une feuille de matériau polymérique et/ou composite pliée en deux de manière à former les deux parois (21) de l'enveloppe (20), afin que l'enveloppe (20) soit hermétique la feuille repliée est au moins partiellement scellée sur le pourtour de l'enveloppe (20), ou par deux feuilles de matériau polymérique et/ou composite formant deux parois (21) de l'enveloppe (20), les deux feuilles étant scellées entre elles sur leur pourtour.

2. Bio-indicateur (1) selon la revendication 1, **caractérisé en ce que** l'enveloppe (20) est entièrement transparente à la lumière selon un spectre de longueur d'onde compris entre 180 nm et 1100 nm, de préférence l'enveloppe (20) est réalisée dans un matériau polymérique et/ou composite possédant des propriétés optiques de transparence à la lumière selon un spectre de longueur d'onde compris entre 180 nm et 1100 nm.

3. Bio-indicateur (1) selon l'une des revendications 1 à 3, **caractérisé en ce que** l'élément support (3) est au moins partiellement hydrophile.

4. Bio-indicateur (1) selon l'une des revendications 1 à 4, **caractérisé en ce que** les micro-organismes (4) sont maintenus en suspension dans une solution aqueuse non saline.

5. Bio-indicateur (1) selon la revendication 4 **caractérisé en ce que** les micro-organismes (4) sont en ensemencés en monocouche selon un dépôt single spot dans un volume déterminé de solution aqueuse non saline.

6. Bio-indicateur (1) selon l'une des revendications 1 à 5 **caractérisé en ce que** le support solide est formé, par au moins une partie d'une face intérieure (26) d'une paroi (21) de l'enveloppe (20), ou par une plaque (30) solide contenue par l'enveloppe (20).

7. Bio-indicateur (1) selon l'une des revendications 1 à 6 **caractérisé en ce que** l'élément support (3) est formé par un matériau qui possède une énergie de surface comprise entre 35 mN/m et 55 mN/m, de préférence, l'élément support (3) est formé par un matériau qui possède une énergie de surface comprise entre 40 mN/m et 50 mN/m, et de préférence l'élément support (3) est formé par un matériau qui possède une énergie de surface comprise entre 43 mN/m et 48 mN/m.

8. Bio-indicateur (1) selon l'une des revendications 1 à 7 **caractérisé en ce qu'**il comporte des moyens de fixation (22) à une surface d'accroche.

9. Procédé de préparation d'un bio-indicateur (1) selon l'une des revendications 1 à 8, **caractérisé en ce qu'**il comporte :
- une étape de stérilisation de l'élément support (3) ;
- une étape d'ensemencement de l'élément support (3) par un nombre déterminé de micro-organismes (4) selon un ensemencement monocouche ;
- une étape de formation de l'enveloppe (20) ; et
- une étape de scellement hermétique de l'enveloppe (20).

10. Procédé de contrôle de l'efficacité d'un système de décontamination par une technologie de décontamination aux rayonnements UV par lumière pulsée, le procédé utilisant un bio-indicateur (1) selon l'une des revendications 1 à 9, **caractérisé en ce qu'**il comporte :
- une étape d'accrochage d'au moins un bio-indicateur (1) qui est solidarisée à un élément prenant part à un processus industrielle soumis à une décontamination à la lumière pulsée ;
- une étape d'irradiation qui consiste à exposer au moins un bio-indicateur (1) positionné sur ledit élément aux rayonnements de la lumière pulsée ; et
- une étape d'analyse de la quantité de micro-organismes (4) détruits et/ou vivants, suite à l'irradiation, l'étape d'analyse comportant une opération de récupération des micro-organismes (4) vivants et une opération de culture des micro-organismes vivants (4) afin de déterminer l'efficacité du système de décontamination.

## Patentansprüche

1. Bioindikator (1) zum Dekontaminieren durch eine UV-Strahlungsdekontaminationstechnologie durch gepulstes Licht, der Bioindikator (1) umfassend einen Behälter (2), der ein Trägerelement (3) einschließt, das eine bestimmte Anzahl von Mikroorganismen (4) trägt, wobei das Trägerelement (3) ein fester Träger ist und die Mikroorganismen (4) auf dem Trägerelement (3) gemäß einer Beimpfung in einer Einzelschicht angeordnet sind, wobei der Behälter (2) eine luftdichte Hülle (20) umfasst, die durch Wände (21) ausgebildet wird, wovon mindestens ein Abschnitt (23) für gepulstes Licht gemäß einem Wellenlängenspektrum zwischen 180 nm und 1100 nm durchlässig ist, wobei dieser durchlässige Abschnitt (23) so gegenüber dem Trägerelement (3) angeordnet ist, dass die Mikroorganismen (4) alle direkt der Strahlung von gepulstem Licht ausgesetzt werden können, wobei die Hülle (20) aus mindestens einer in zwei Hälften gefalteten Folie aus Polymer- und/oder Verbundmaterial, um die zwei Wände (21) der Hülle (20) so auszubilden, dass die Hülle (20) luftdicht ist, wobei die gefaltete Folie mindestens teilweise um den Umfang der Hülle (20) versiegelt ist, oder aus zwei Folien aus Polymer- und/oder Verbundmaterial ausgebildet ist, die zwei Wände (21) der Hülle ausbilden (20), wobei die zwei Folien an ihrem Umfang miteinander versiegelt sind.

2. Bioindikator (1) nach Anspruch 1, **dadurch gekennzeichnet, dass** die Hülle (20) für Licht gemäß einem Wellenlängenspektrum zwischen 180 nm und 1100 nm vollständig durchlässig ist, vorzugsweise die Hülle (20) aus einem Polymer- und/oder Verbundmaterial hergestellt ist, das optische Eigenschaften einer Durchlässigkeit für Licht gemäß einem Wellenlängenspektrum zwischen 180 nm und 1100 nm besitzt.

3. Bioindikator (1) nach einem der Ansprüche 1 bis 3,
**dadurch gekennzeichnet, dass** das Trägerelement (3) mindestens teilweise hydrophil ist.

4. Bioindikator (1) nach einem der Ansprüche 1 bis 4,
**dadurch gekennzeichnet, dass** die Mikroorganismen (4) in einer nicht salzhaltigen wässrigen Lösung in Suspension gehalten werden.

5. Bioindikator (1) nach Anspruch 4, **dadurch gekennzeichnet, dass** die Mikroorganismen (4) gemäß einer Single-Spot-Abscheidung in ein bestimmtes Volumen der nicht salzhaltigen wässrigen Lösung in der Einzelschicht eingeimpft sind.

6. Bioindikator (1) nach einem der Ansprüche 1 bis 5,
**dadurch gekennzeichnet, dass** der feste Träger durch mindestens einen Teil einer Innenfläche (26) einer Wand (21) der Hülle (20) oder durch eine feste Platte (30) ausgebildet ist, die durch die Hülle (20) enthalten ist.

7. Bioindikator (1) nach einem der Ansprüche 1 bis 6,
**dadurch gekennzeichnet, dass** das Trägerelement (3) aus einem Material ausgebildet ist, das eine Oberflächenenergie zwischen 35 mN/m und 55 mN/m besitzt, vorzugsweise das Trägerelement (3) aus einem Material ausgebildet ist, das eine Oberflächenenergie zwischen 40 mN/m und 50 mN/m aufweist, und vorzugsweise das Trägerelement (3) aus einem Material ausgebildet ist, das eine Oberflächenenergie zwischen 43 mN/m und 48 mN/m besitzt.

8. Bioindikator (1) nach einem der Ansprüche 1 bis 7,
**dadurch gekennzeichnet, dass** er Mittel (22) zum Fixieren an einer Befestigungsoberfläche aufweist.

9. Verfahren zum Herstellen eines Bioindikators (1) nach einem der Ansprüche 1 bis 8, **dadurch gekennzeichnet, dass** es aufweist:
- einen Schritt eines Sterilisierens des Trägerelements (3);
- einen Schritt des Beimpfens des Trägerelements (3) mit einer bestimmten Anzahl von Mikroorganismen (4) gemäß einer einschichtigen Beimpfung;
- einen Schritt des Ausbildens der Hülle (20); und
- einen Schritt des luftdichten Versiegelns der Hülle (20).

10. Verfahren zum Überwachen der Wirksamkeit eines Systems zum Dekontaminieren durch eine UV-Strahlungsdekontaminationstechnologie durch gepulstes Licht, wobei das Verfahren einen Bioindikator (1) nach einem der Ansprüche 1 bis 9 verwendet, **dadurch gekennzeichnet, dass es** aufweist:
- einen Schritt des Befestigens mindestens eines Bioindikators (1), der mit einem Element fest verbunden ist, das an einem industriellen Prozess teilnimmt, das einer Dekontamination mit gepulstem Licht unterzogen wird;
- einen Schritt eines Bestrahlens, der darin besteht, mindestens einen Bioindikator (1), der auf dem Element positioniert ist, Strahlung von gepulstem Licht auszusetzen; und
- einen Schritt zum Analysieren der Menge der zerstörten und/oder lebenden Mikroorganismen (4) nach der Bestrahlung, wobei der Analyseschritt einen Vorgang zum Rückgewinnen der lebenden Mikroorganismen (4) und einen Vorgang zum Kultivieren lebender Mikroorganismen (4) aufweist, um die Wirksamkeit des Dekontaminationssystems zu bestimmen.

## Claims

1. Bio-indicator (1) for decontamination by UV-radiation decontamination technology using pulsed light, the bio-indicator (1) comprising a container (2) enclosing a support element (3) which supports a specific number of micro-organisms (4), the support element (3) is a solid support and the micro-organisms (4) are arranged on the support element (3) in accordance with monolayer seeding, the container (2) comprising a hermetic shell (20) which is formed by walls (21), at least one portion (23) of which is transparent to pulsed light in a wavelength spectrum of between 180 nm and 1100 nm, this transparent portion (23) being arranged opposite the support element (3) so that all the micro-organisms (4) can be directly exposed to the pulsed light radiation, the shell (20) being formed by at least one sheet of polymeric and/or composite material which is folded in two, thereby forming the two walls (21) of the shell (20), the folded sheet being at least partially sealed around the edge of the shell (20) so that the shell (20) is hermetic, or formed by two sheets of polymeric and/or composite material forming two walls (21) of the shell (20), the two sheets being sealed together around the edge thereof.

2. Bio-indicator (1) according to claim 1, **characterized in that** the shell (20) is completely transparent to light in a wavelength spectrum of between 180 nm and 1100 nm, and the shell (20) is preferably made of a polymeric and/or composite material with optical properties of transparency to light in a wavelength spectrum of between 180 nm and 1100 nm.

3. Bio-indicator (1) according to one of claims 1 to 3, **characterized in that** the support element (3) is at least partially hydrophilic.

4. Bio-indicator (1) according to one of claims 1 to 4, **characterized in that** the micro-organisms (4) are held in suspension in a non-saline aqueous solution.

5. Bio-indicator (1) according to claim 4, **characterized in that** the micro-organisms (4) are seeded in a monolayer as a single spot deposit in a specific volume of non-saline aqueous solution.

6. Bio-indicator (1) according to one of claims 1 to 5, **characterized in that** the solid support is formed by at least part of an inner face (26) of a wall (21) of the shell (20), or by a solid plate (30) contained by the shell (20).

7. Bio-indicator (1) according to one of claims 1 to 6, **characterized in that** the support element (3) is formed by a material which has a surface energy of between 35 mN/m and 55 mN/m, the support element (3) is preferably formed by a material which has a surface energy of between 40 mN/m and 50 mN/m, and preferably the support element (3) is formed by a material which has a surface energy of between 43 mN/m and 48 mN/m.

8. Bio-indicator (1) according to one of claims 1 to 7, **characterized in that** it comprises means (22) for attaching to a coupling surface.

9. Method for preparing a bio-indicator (1) according to one of claims 1 to 8, **characterized in that** it comprises:
- a step of sterilizing the support element (3);
- a step of seeding the support element (3) with a specific number of micro-organisms (4) in accordance with monolayer seeding;
- a step of forming the shell (20); and
- a step of hermetically sealing the shell (20).

10. Method for monitoring the effectiveness of a system for decontamination by UV-radiation decontamination technology using pulsed light, the method using a bio-indicator (1) according to one of claims 1 to 9, **characterized in that** it comprises:
- a step of coupling at least one bio-indicator (1) which is secured to an element involved in an industrial process subjected to pulsed light decontamination;
- a step of irradiation which consists in exposing at least one bio-indicator (1) positioned on said element to pulsed light radiation; and
- a step of analyzing the quantity of micro-organisms (4) destroyed and/or alive following the irradiation, the analysis step comprising an operation for recovering the living micro-organisms (4) and an operation for culturing the living micro-organisms (4) in order to determine the effectiveness of the decontamination system.
